# EUROPEAN PATENT SPECIFICATION

(11) **EP 4 076 337 B1**
(45) Date of publication and mention of the grant of the patent: **22.07.2026**
(21) Application number: 20815839.4
(22) Date of filing: 02.12.2020
(51) Int. Cl.: A61K 8/02, A61K 8/35, A61Q 1/06

(54) **DIALIPHATIC KETONE MIXTURES, COMPOSITIONS COMPRISING SAME AND USE THEREOF**
DIALIPHATISCHE KETONMISCHUNGEN, ZUSAMMENSETZUNGEN DAMIT UND VERWENDUNG DAVON
MÉLANGES DE CÉTONES DIALIPHATIQUES, COMPOSITION LES COMPRENANT ET LEUR UTILISATION

(30) Priority: 17.12.2019 EP 19216796
(43) Date of publication of application: 26.10.2022
(73) Proprietor: SYENSQO SA, 1130 Brussels (BE)
(72) Inventor: DERRIEN, Elie, 69530 Brignais (FR); MASTROIANNI, Sergio, 69006 Lyon (FR)
(74) Representative: Valentino, Cédric
(86) International application number: PCT/EP2020/084298
(87) International publication number: WO 2021/122005

(56) References cited:
- WO-A1-2018/033607
- JP-A- 2003 113 033
- JP-A- 2003 113 035

## Description

The present invention relates to mixtures of dialiphatic ketones, compositions comprising such mixtures and the use of the mixtures and compositions for cosmetic purposes.

Dialiphatic ketones, in particular long chain ketones obtained from fatty acids, fatty acid derivatives or fatty acid cuts or mixtures thereof, are useful intermediates for the synthesis of a variety of end products such as alcohols, twin-tail amines and internal olefins, to mention only a few examples.

WO 2018/033607 relates to a process for synthesizing internal ketones by decarboxylative ketonization of fatty acids or fatty acid mixtures. In working example 1 a ketone mixture is described comprising 68.5 wt% of dialiphatic ketones with 15 to 25 carbon atoms, 28.4 wt% of dialiphatic ketones with 27 to 31 carbon atoms and 2.5 wt% of dialiphatic ketones with 33 carbon atoms or more. Working example 2 describes a similar mixture, comprising 2.2 wt% of dialiphatic ketones having 33 or more carbon atoms. This application also discloses certain products which can be obtained from the dialiphatic ketones.

WO 2018/087179 relates to a process for the decarboxylative ketonization of fatty acids, fatty acid derivatives or mixtures thereof in the liquid phase with metal compounds as catalyst. Fatty acid cuts are used in the working examples as starting material and no data are provided on the composition of the ketone mixture obtained.

Cosmetic products are widely used for a variety of applications, including e.g. stick-shaped cosmetic products, also known as cosmetic sticks. Such sticks are uses as coloured lipsticks, moisturizing sticks, deodorizing sticks or depilatory sticks and a variety of other applications to name only a few products of this type.

08/079478 discloses a composition for imparting an unctuous film to the lips which comprises a first and second wax component having melting points respectively below and above a certain temperature. Among the waxes with a melting point exceeding said defined temperature, stearone (18-pentatriacontanone) is mentioned as an example.

JP 2003/113035 discloses a stick shaped cosmetics comprising solid dialkyl ketones of general formula R¹-C(=O)-R² wherein R¹ and R² are the same or different any may be alkyl groups having 10 to 20 carbon atoms. Symmetrical ketones are mentioned as preferred and stearone and laurone (12-tricosanone) as well as mixtures thereof are used in the working examples. The compositions can also contain natural waxes such as carnauba or candelilla wax.

2003/113033 relates to water-in oil type cosmetics characterized by a content of solid dialkylketones of the same formula as given in JP 2003/113035. Symmetrical ketones are mentioned as preferred and stearone and laurone (12-tricosanone) and mixtures thereof are used in the working examples

JP 2003/113034 relates to oil in water type cosmetics characterized by a content of solid dialkylketones of the same formula as given in JP 2003/113035. Symmetrical ketones are mentioned as preferred and stearone and laurone (12-tricosanone) are used in the working examples.

JP2003/113041 relates generally to cosmetics characterized in containing dialkylketones of the same formula as given in JP 2003/113035. Symmetrical ketones are mentioned as preferred and stearone and laurone (12-tricosanone) are used in the working examples.

JP2003/286123 relates to oily cosmetics containing solid dialkylketones as described in JP 2003/111035 in combination with a liquid oil. Symmetrical ketones are mentioned as preferred and stearone and laurone (12-tricosanone) as well as mixtures thereof are used in the working examples. The compositions can also contain natural waxes such as carnauba or candelilla wax.

The compositions described in the prior art comprising mixtures of dialkyl ketones are not fully satisfactory in terms of their property profile, which does not fulfill to the desired degree the property profile considered advantageous especially for cosmetic applications.

It was thus an object of the present invention to provide improved mixtures and compositions comprising dialiphatic ketones.

This object is achieved in accordance with the mixtures in accordance with claim 1 and in accordance with the compositions in accordance with claim 11.

Preferred embodiments of the present invention are set forth in the dependent claims and in the detailed specification hereinafter.

The ketone mixture M in accordance with the present invention comprises different dialiphatic ketones as follows:
a) from 25 to 73 wt% based on the total weight of ketone mixture M, of at least one dialiphatic ketone K1 comprising from 15 to 25 carbon atoms,
b) from 2 to 40 wt%, preferably from 4 to 35 wt% and even more preferably from 5 to 30 wt%, based on the total weight of ketone mixture M, of at least one dialiphatic ketone K2 comprising from 26 to 32 carbon atoms and,
c) from 25 to 90 wt%, preferably from 40 to 80 wt% and more preferably from 45 to 75 wt%, based on the total weight of the ketone mixture M, of at least one dialiphatic ketone K3 comprising from 33 to 47 carbon atoms.

The term "ketone mixture M" as used herein denotes a mixture consisting essentially of several ketones ; preferably, the ketone mixture M consists of, i.e. consists entirely of, several ketones. The ketone mixture M comprises the dialiphatic ketones K1, K2 and K3 in the amounts as defined in claim 1. In addition to the dialiphatic ketones K1, K2 and K3, the ketone mixture M may also comprise other ketones, such as e.g. dialiphatic ketones with less than 15 or with more than 47 carbon atoms or ketones other than dialiphatic ketones or a mixture thereof. The ketone mixture M in accordance with the present invention is essentially free of any components other than ketones and preferably is free, i.e. is entirely free, of any components other than ketones.

Thus, the ketone mixture M in accordance with the present invention may contain one or more other ketone(s) besides ketones K1 to K3.

Furthermore, the ketone mixture M may contain more than one ketone K1, K2 or K3, i.e. more than one ketone comprising the carbon numbers as defined for ketones K1, K2 and K3. Thus, K1, K2 and K3 only denote the number of carbon atoms of the respective ketones but does not limit the mixture to only one representative of each of K1, K2 and K3.

In accordance with a preferred embodiment, the dialiphatic ketones K1, K2 and K3 constitute at least 95 wt%, preferably at least 98 wt% of the ketone mixture), even more preferably the ketone mixture M consists of ketones K1, K2 and K3.

The dialiphatic ketones in the ketone mixture M of the present invention preferably have a melting point of at least 40°C, preferably in the range of from 40 to 200°C.

In accordance with a preferred embodiment the dialiphatic ketones of ketone mixture M have individual melting points in the range of from 40 to 100°C, in particular from 55 to 95°C and even more preferred of from 65 to 95 C. The ketone mixture can have only one melting point if the mixture is homogenous. In this case the single melting point of the ketone mixture M is preferably in the range of from 40 to 100°C, preferably in the range of from 50 to 95°C.

Ketone mixtures obtained from fatty acid cuts may frequently have melting points below the melting point of the lowest melting individual ketone in the mixture. It may thus be that dialiphatic ketone mixtures M in accordance with the present invention have single melting points below 40°C if the ketone mixture M contains primarily dialiphatic ketones having less than 23 carbon atoms.

While it is not mandatory that the ketone mixture M as such has a melting point of at least 40°C, preferably of from 40 to 100°C, it is preferred that the melting point of the ketone mixture M is in the said range.

The ketone mixture M comprises different dialiphatic ketones. It can comprise at least three different dialiphatic ketones, possibly more than three different dialiphatic ketones, in particular at least four or at least five different dialiphatic ketones. It may contain up to 20, in particular up to 12 different dialiphatic ketones.

It has surprisingly been found that ketone mixtures M comprising ketones in the weight ratios given above possess beneficial properties, in particular for use in cosmetics.

Preferred dialiphatic ketones are represented by general formula R-C(=O)-R', wherein R and R', which may be the same or different, represent a saturated or unsaturated, straight chain or branched aliphatic radical with of from 5 to 24, preferably from 6 to 20 and even more preferred from 7 to 18 carbon atoms, preferably a saturated aliphatic group and most preferably an alkyl group of from 5 to 24, preferably from 6 to 20 and even more preferred from 7 to 18 carbon atoms.

Examples of suitable radicals R and R' are octyl (C₈), nonyl (C₉), decyl (C₁₀), undecyl (C₁₁), dodecyl (C₁₂), tridecyl (C₁₃) tetradecyl (C₁₄), hexadecyl (C₁₆), octadecyl (C₁₈) and eicosyl (C₂₀), to name only some examples.

Dialiphatic ketones K1, if present in the ketone mixture M of the present invention, comprise from 15 to 25, preferably from 19 to 24 carbon atoms.

Dialiphatic ketone K1 comprises preferably at least 19 carbon atoms, more preferably at least 21 carbon atoms.

The dialiphatic ketone K1 is preferably of formula R¹-C(=O)-R¹, wherein
R¹ and R^{'1} are aliphatic groups. R¹ and R^{'1} are preferably alkyl or alkenyl, more preferably alkyl. R¹ and R^{'1} are preferably linear aliphatic groups, in particular linear alkyl or alkenyl groups.

R¹ and R^{'1} comprise preferably at least 4 carbon atoms, more preferably at least 6 carbon atoms, still more preferably at least 8 carbon atoms, even more preferably at least 10 carbon atoms and the most preferably at least 11 carbon atoms.

R¹ and R^{'1} comprise preferably at most 20 carbon atoms, more preferably at most 18 carbon atoms, still more preferably at most 16 carbon atoms, even more preferably at most 14 carbon atoms and the most preferably at most 13 carbon atoms.

Good results are obtained when the dialiphatic ketone K1 comprises from 21 to 25 carbon atoms and is of formula R¹-C(=O)-R¹ wherein R¹ and R¹ are linear C₁₁-C₁₃-alkyl.

No dialiphatic ketone K1 may be present in the ketone mixture M. Alternatively and preferably, the ketone mixture M can comprise the dialiphatic ketone K1 in an amount of up to 72 wt.%, based on the total weight of the ketone mixture M.

The weight fraction of the dialiphatic ketone K1, based on the total weight of the ketone mixture M, is preferably of at most 55 wt.%, more preferably at most 40 wt. %, still more preferably at most 35 wt. %, even more preferably at most 30 wt. %.

Dialiphatic ketones K2 present in the ketone mixture M of the present invention comprise from 26 to 32, preferably from 27 to 31 carbon atoms.

Dialiphatic ketone K2 is preferably of formula R²-C(=O)-R^{'2} wherein R² and R^{'2} are aliphatic groups. R² and R^{'2} are preferably alkyl or alkenyl, more preferably alkyl. R² and R^{'2} are preferably linear aliphatic groups, in particular linear alkyl or alkenyl groups.

R² and R^{'2} comprise preferably at least 6 carbon atoms, more preferably at least 10 carbon atoms, still more preferably at least 12 carbon atoms.

R² and R^{'2} comprise preferably at most 24 carbon atoms, more preferably at most 20 carbon atoms, still more preferably at most 18 carbon atoms.

Good results are obtained when the dialiphatic ketone K₂ is of formula
R²-C(=O)-R^{2'} wherein R² and R^{2'} are linear C₁₂-C₁₈ alkyl.

The ketone mixture M comprises the dialiphatic ketone K2 in an amount of from 2 wt. % to 40 wt. %, based on the total weight of the ketone mixture M.

The weight fraction of the dialiphatic ketone K2, based on the total weight of the ketone mixture M, is preferably of at least 3.5 wt.%, more preferably at least 5 wt. %, and may be in some embodiments of at least 10 wt. % or at least 15 wt. %.

The weight fraction of the dialiphatic ketone K2, based on the total weight of the ketone mixture M, is preferably of at most 35 wt.%, more preferably at most 30 wt. %, still more preferably at most 25 wt. %.

Dialiphatic ketones K3 present in the ketone mixture M of the present invention comprise from 33 to 47, preferably from 33 to 39 carbon atoms.

Dialiphatic ketone K3 comprises preferably at most 43 carbon atoms, more preferably at most 39 carbon atoms, still more preferably at most 37 carbon atoms.

The dialiphatic ketone K3 is preferably of formula R³-C(=O)-R^{'3}, wherein
R³ and R^{'3} are aliphatic groups. R³ and R^{'3} are preferably alkyl or alkenyl, more preferably alkyl. R³ and R^{'3} are preferably linear aliphatic groups, in particular linear alkyl or alkenyl groups.

R³ and R^{'3} comprise preferably at most 40 carbon atoms, more preferably at most 30 carbon atoms, still more preferably at most 24 carbon atoms, even more preferably at most 21 carbon atoms and the most preferably at most 19 carbon atoms.

R³ and R^{'3} comprise preferably at least 8 carbon atoms, more preferably at least 12 carbon atoms, still more preferably at least 16 carbon atoms, even more preferably at least 17 carbon atoms and the most preferably at least 18 carbon atoms.

Good results are obtained when the dialiphatic ketone K₃ comprising from 33 to 37 carbon atoms and is of formula R³-C(=O)-R^{'3} wherein R³ and R^{'3} are linear C₁₇-C₁₉ alkyl.

The mixture M comprises the dialiphatic ketone K₃ in an amount of from 25 wt. % to 90 wt. %, based on the total weight of the mixture M.

The weight fraction of the dialiphatic ketone K₃, based on the total weight of the mixture M, is preferably of at least 40 wt.%, more preferably of at least 45 wt. %, still more preferably at least 50 wt. %, and may be in some embodiments of at least 60 wt. % or at least 65 wt. %.

The weight fraction of the dialiphatic ketone K₃, based on the total weight of the ketone mixture M, is preferably of at most 80 wt.%, more preferably at most 75 wt. %, still more preferably at most 70 wt. %.

A ketone mixture M which has been found advantageous for certain uses, including cosmetic applications, comprises at least laurone (23 C-atoms), stearone (35 C-atoms) and heptadecyl undecyl ketone (29 C-atoms), optionally in combination with further ketones.

In accordance with still another preferred embodiment the dialiphatic ketones in the ketone mixtures M of the present invention are obtained by decarboxylative ketonization of fatty acids, fatty acid derivatives or mixtures thereof in the liquid phase with metal compounds as catalyst.

Preferably, the fatty acid is selected from the group consisting of isostearic acids, caprylic acid, capric acid, lauric acid, myristic acid, palmitic acid, stearic acid, arachidic acid, behenic acid, lignoceric acid, cerotic acid or mixtures thereof.

The term fatty acids derivative refers to anhydrides made by the condensation of 2 fatty acids or to esters made by the condensation of fatty acids with alcohols.

Suitable fatty acid derivatives are esters and anhydrides of fatty acids, but the use of the free fatty acids as such is generally preferred. The esters or anhydrides in the course of the reaction are converted to the acids which then react with the metal or the metal compound.

The fatty acid or fatty acid derivatives can be used in the form of so called fatty acid or fatty acid derivatives cuts which may be obtained by the hydrolysis or alcoholysis of different natural fats and oils. Accordingly these cuts may contain various amounts of different linear fatty acids or linear fatty acid derivatives with different chain lengths. Just by way of example, fatty acid cuts comprising mainly C₈-C₁₈ fatty acids, preferably mainly C₁₂-C₁₈ fatty acids may be mentioned here. The skilled person is well aware of fatty acid cuts obtainable from various sources and will select the best suitable starting materials based on the desired ketones. The composition of fatty acid cuts may vary over a great range and the ketone mixture obtained from such fatty acid cut thus may also differ in the weight percentages of the individual ketones in the mixture (depending on the composition of the fatty acid cut from which the ketone mixture is obtained).

The fatty acid may be or include oleic acid, linoleic acid, linolenic acid, erucic acid, palmitoleic acid or a mixture thereof.
When starting from a single fatty acid, a symmetrical ketone is obtained as the reaction product; when starting from a cut of fatty acids as described above all the ketones formed by the combination of the different alkyl groups of the starting acids are obtained and the distribution of the different mixed ketones generally follows a statistical binomial law. The reaction equation can be summarized as follows:

Rₙ-COOH + Rₘ-COOH → Rₙ₋C(=O)-Rₘ + CO₂ + H₂O

wherein Rₙ and Rₘ represent the aliphatic groups of the fatty acids present in the cut. It is well apparent that e.g. if three different acids are present, a total of six different ketones may be formed; three symmetrical ketones wherein Rₙ and Rₘ are identical and three unsymmetrical ketones with different groups Rₙ and Rₘ.

According to a preferred embodiment, the ketone mixtures M of the present invention comprise a mixture of dialiphatic ketones obtained by decarboxylative ketonization of a fatty acid cut comprising at least two different fatty acids, preferably with from 8 to 18, even more preferably with from 12 to 18 carbon atoms. If the weight ratio of the different ketones obtained from decarboxylative ketonization of a fatty acid cut is outside the ranges as defined in claim 1 and in the specification hereinbefore, additional amounts of ketones can be added to obtain a ketone mixture M in accordance with the present invention. A respective product obtained from a fatty acid cut is hereinafter referred to as **IK** C₂₃-C₃₅. IK stands for internal ketone and C₂₃-C₃₅ in the designation indicates the total number of carbon atoms in the individual ketones present in the mixture. IK C₂₃-₃₅ used in the working examples is a mixture of at least three dialiphatic ketones each comprising of from 23 to 35 carbon atoms. This mixture can comprise e.g. at least stearone (symmetrical dialiphatic ketone with 35 C-atoms), laurone (symmetrical dialiphatic ketone with 23 C-atoms) and heptadecyl undecyl ketone (which is the mixed dialiphatic ketone obtained from the decarboxylative ketonization of a mixture of lauric acid and stearic acid comprising 29 C atoms). IK C₂₃-C₃₅ as used in the examples comprises other dialiphatic ketones in addition to the three mentioned ketones. The weight ratio of ketones K1, K2 and K3 is outside the ranges as in the present invention so that IK C₂₃-₃₅ is blended with other ketones to arrive at the ketone mixtures M of the present invention. In particular, laurone and/or stearone can be added to IK C₂₃-C₃₅ to arrive at the ketone mixtures M of the present invention.

The conversion of fatty acids into respective ketones by decarboxylative ketonization is a well-known process which is also commercially used and has been described in the literature.

The process disclosed in these references can be carried out in the gas phase at temperatures usually exceeding 350°C and usually above 400°C for fatty acids in the presence of catalytic amounts of metal oxide compounds (e.g. MgO, ZrO₂, Al₂O₃, CeO₂, MnO₂, TiO₂).

The processes disclosed in EP 3 292 097 and WO 2018/087179 offer an easy access to internal ketones which can be used as ketones in the ketone mixtures M in accordance with the present invention.

The ketones may be separated from the reaction mixture by convenient and economic processes and the catalytic material can be used for several catalytic cycles without significant deterioration of catalytic activity.

Dialkyl ketones may also be obtained by dry-distilling salts, in particular alkaline earth metal salts of higher fatty acids. Respective processes have been described in the literature and are known to the skilled person.

Another embodiment of the present invention relates to compositions comprising the ketone mixtures M as defined hereinbefore in combination with further components other than ketones.

Such compositions preferably comprise the ketone mixtures M of the present invention as defined hereinbefore and additionally one or more components selected from
d) animal oils, vegetable oils, or synthetic oils, or from solid oils, semisolid oils, liquid oils, or volatile oils,
e) hydrocarbons, in particular fluid paraffins
f) waxes,
g) esters,
h) alcohols,
i) silicones,
j) fluorine based oils, and
k) lanolin.

Furthermore, the compositions in accordance with the present invention may comprise, in particular if the ketone mixture M is obtained by decarboxylative ketonization of fatty acids, fatty acid derivatives or mixtures thereof, and the reaction product is used without purification, certain amounts of unreacted starting materials or by-products. In many cases these compounds constitute less than 5, often less than 3 percent of the weight of the composition.

The weight percentage of the ketone mixture M of the present invention in the compositions of the present invention is not subject to particular limitations and the skilled person will adjust the content based on the need of the specific application based on his professional knowledge. Weight percentages in the range from 1 to 40 wt%, preferably of from 3 to 30 wt%, even more preferably from 4 to 20 wt%, based on the weight of the composition have been found advantageous in certain cases.

The compositions of the present invention may be water based (if the composition is of the oil-in water type) or oil based (if the composition is oil based). The composition may also contain further components such as additives used in e.g. standard cosmetics for purposes such as adjusting touch or imparting an emollient feel.

The oils d) used in the compositions of the present invention may be of any origin such as animal oil, vegetable oil, or synthetic oil, or from solid oil, semisolid oil, liquid oil, or volatile oil, and examples are oils such as, hydrogenated oils, ester oils or fluorine-based oils.

Specific examples of oils d), hydrocarbons e) and waxes f) are fluid paraffin, squalane, vaseline, paraffin wax, ceresin wax, microcrystalline wax, montan wax, or Fischer-Tropsch wax, an oil or fat such as Japan wax, olive oil, castor oil, jojoba oil, mink oil, or macadamia nut oil, or waxes f) such as beeswax, carnauba wax, candelilla wax, or spermaceti.

Examples for esters g) are cetyl isooctanoate, isopropyl myristate, isopropyl palmitate, octyldodecyl myristate, glyceryl trioctanoate, diglyceryl diisostearate, diglyceryl triisostearate, glyceryl tribehenate, pentaerythritol rosin ester, neopentyl glycol dioctatanoate, cholesterol fatty acid ester, or di(cholesterol/behenyl/octyldodecyl) N-lauroyl-L-glutamate.

Examples for alcohols h) are higher alcohols such as stearyl alcohol, cetyl alcohol, lauryl alcohol, oleyl alcohol, isostearyl alcohol, or behenyl alcohol.

Exemplary silicones i) are low molecular weight dimethylpolysiloxane, high molecular weight dimethylpolysiloxane, methylphenylpolysiloxane, decamethylcyclopentasiloxane and octamethylcyclotetrasiloxane and . fluorine-modified silicones.

Suitable fluorine based oils j) are perfluoropolyether, perfluorodecane, or perfluorooctane, and exemplary lanolin derivatives k) lanolin acetate, isopropyl lanolin fatty acid, or lanolin alcohol; one or more of which may be used.

The content of oils is preferably 0.1-80 wt% , based on the entire weight % of the composition.

In accordance with a particularly preferred embodient of the present invention , the cosmetic composition comprises a combination of a ketone mixture M in accordance with the present invention as described above with natural waxes f) such as carnauba wax or candelilla wax to name only two examples. Other examples for waxes f) are polyolefin wax, Ozokerite, microcristalline wax, paraffin wax, Fischer-Tropsch wax, montan wax, Ceresin, beeswax, jojoba wax, rice bran wax, sugarcane wax, japan wax, tea wax, sunflower wax, berry wax, myrica fruit wax, laurel wax, lanolin, spermaceti, fatty ester wax, fatty acid wax.

If such mixtures are used, the weight ratio of the natural waxes to the mixture of ketones is not subject to particular limitations and is preferably in the range of from 5:95 to 95:5, more preferably in the range of from 10:90 to 90:10.

Furthermore, the compositions of the present invention may contain a powder for the purpose of imparting a cosmetic effect or an ultraviolet protective effect and purposes such as adjusting touch. Examples of the powder used in this case are an inorganic extender pigment such as talc, kaolin, sericite, mica, montmorillonite, bentonite, hectorite, saponite, organic modified clay minerals, calcium carbonate, aluminium silicate, magnesium silicate, boron nitride, cerium oxide, zirconium oxide, aluminium-magnesium silicate, calcium silicate, or anhydrous silica, an inorganic colour pigment such as titanium oxide, iron-containing titanium oxide, zinc oxide, aluminium oxide, barium sulphate, red iron oxide, yellow iron oxide, black iron oxide, ultramarine, or Prussian blue, a photoluminescent pigment such as mica titanium, iron oxide mica titanium, or bismuth oxychloride, a lamé agent such as polyethylene terephthalate-aluminium-epoxy laminated powder or polyethylene terephthalate-polyolefin laminated powder, an inorganic colour pigment such as tar dye or a natural dye, a silicone resin powder such as organopolysiloxane hardened powder or polymethyl silsesquioxane powder, and an organic powder such as nylon powder, polymethyl methacrylate, silk powder, polyethylene powder, crystalline cellulose, magnesium stearate, or N-acyllysine; one or more may be selected from among these as required. The particle size and shape of these powders are not specifically limited. A compound of two or more of these powders may be used, and the surface may be treated by a conventional method with a fluorine compound, a silicone-based compound, a metallic soap, a wax, a fat or oil, a hydrocarbon, a surfactant, an amino acid derivative, a water-soluble polymer, or the like. The content of powders in the cosmetic compositions of the present invention is preferably 0.1-40 wt%, based on the weight of the composition.

As a further component, the composition of the present invention may contain a surfactant as an emulsifier or an emulsification auxiliary agent.

For the purpose of the present invention, the term "surfactant" means a compound that, when present in water, lowers the surface tension of water. Surfactants may act e.g. as wetting agents, foaming agents, and dispersants.

Examples of such a surfactant are an anionic surfactant, a cationic surfactant, an amphoteric surfactant, and a non-ionic surfactant, one or more of which may be used. Specific examples of an anionic surfactant are a fatty acid such as stearic acid, lauric acid, myristic acid, behenic acid, isostearic acid, oleic acid, or 12-hydroxystearic acid, a fatty acid soap formed by an alkali such as sodium, potassium, or triethanolamine, an acylglutamate salt, an alkyl phosphate, and a polyoxyalkylene adduct alkyl phosphate salt. Examples of a cationic surfactant are an alkylamine salt and an alkyl quaternary ammonium salt. Examples of an amphoteric surfactant are N,N-dimethyl-N-alkyl-N-carboxymethyl ammonium betaine, N,N-dialkylaminoalkylene carboxylic acid, N,N,N-trialkyl-N-sulphoalkylene ammonium betaine, N,N-dialkyl-N,N-bis(polyoxyethylene sulphate)ammonium betaine, 2-alkyl-1-hydroxyethyl-1-carboxymethylimidazolinium betaine, and a phospholipid such as lecithin. Examples of a non-ionic surfactant are sugar fatty acid esters such as glycerine fatty acid ester and alkylene glycol adducts thereof, polyglycerine fatty acid ester and alkylene glycol adducts thereof, propylene glycol fatty acid ester and alkylene glycol adducts thereof, sorbitan fatty acid ester and alkylene glycol adducts thereof, sorbitol fatty acid ester and alkylene glycol adducts thereof, polyalkylene glycol fatty acid ester, polyoxyalkylene alkyl ether, glycerine alkyl ether, polyoxyethylene alkyl phenyl ether, polyoxyethylene hydrogenated castor oil, alkylene glycol adduct of lanolin, dextrin fatty acid ester, sucrose fatty acid ester, or starch fatty acid ester, and polyoxyalkylene-modified organosiloxane. The content of surfactants in the composition, if present, of the present invention varies depending on the type of surfactant and the quantity and nature of the oils, but generally is preferably 0.01-20%, based on the weight of the composition.

A first preferred class of surfactants are the so called "poly(alkylene oxide)s". Poly(alkylene oxide)s are polymers essentially all or all the repeating units of which comply with general formula -r-O- wherein r represents a divalent alkylene group having from 2 to 10 carbon atoms. Such poly(alkylene oxide)s may be terminated by a hydroxyl group. Particularly suitable poly(alkylene oxide)s are those wherein r has from 2 to 4 carbon atoms, preferably from 2 to 3 carbon atoms, more preferably 2 carbon atoms. The poly(alkylene oxide)s may be either linear or branched. Linear poly(alkylene oxides) are generally preferred.

Specific examples of suitable poly(alkylene oxide)s include polyoxyalkylene polyols, such as polyoxyethylene glycol (also known as poly(ethylene glycol) or poly(ethylene oxide), polyoxyethylene triol, polyoxyethylene tetraol, polyoxypropylene glycol (also commonly referred to as poly(propylene glycol) or poly(propylene oxide), polyoxypropylene triol, polyoxypropylene tetraol, polyoxybutylene glycol, polyoxypentane glycol, polyoxyhexane glycol, polyoxyheptane glycol, and polyoxyoctane glycol. These polymers may be used either individually or in combinations of two or more; for example, it can be cited random copolymers of ethylene oxide and propylene oxide, and poly(ethylene oxide)-poly(propylene oxide) block copolymers.

The molecular weight of the poly(alkylene oxide)s may cover a wide range. In certain cases poly(alkylene oxide)s having a number average or weight average molecular weight of at least 20,000, preferably at least 200,000 and even more preferably at least 1,000,000 are advantageous. In other cases, average molecular weights of at most 20,000, preferably at most 10,000 and even more preferably at most 1000 are useful. The molecular weight of the suitable poly(alkylene oxides) may also be optimized in view of the specific application case. For example, a methoxy-terminated poly(ethylene oxide) having a number average or a weight average molecular weight of at most 2,000 may be used. Copolymers comprising oxyethylene and oxypropylene units in random or block distribution may also be suitable and respective products are commercially available e.g. under the tradename Pluronics^{®} from BASF.

Besides the components described earlier, the composition of the present invention may contain components e.g. those generally used in cosmetics within a quantitative and qualitative range that does not impair the effects of the present invention; for example, an aqueous component, a water-soluble polymer, an aqueous gelling agent such as bentonite, hectorite, or smectite, a copolymer such as alkyl (meth)acrylate, (meth)acrylic acid, or vinyl polyacetate, a polymer emulsion of a homopolymer dispersed in an aqueous solvent, an oil gelling agent such as aluminium isostearate, calcium stearate, an organic modified clay mineral, or partially cross-linked organopolysiloxane, an ultraviolet absorber such as 2-ethylhexyl paramethoxycinnamate, ethyl paraaminobenzoate, 4-tert-butyl-4'-methoxydibenzoylmethane, or oxybenzone, an antioxidant, a preservative, a vitamin such as vitamin A, B6, B12, C, or E, a chelating agent, a beauty ingredient such as rosemary extract, camisole extract, carrot extract, swertia japonica extract, catechin, a catechin derivative, Glycyrrhiza extract, mulberry bark extract, hops extract, collagen, hyaluronic acid, a hyaluronic acid derivative, trehalose, or aloe extract, and a refreshing agent and fragrance such as menthol, camphor, or sorbitol.

The aqueous component, if present, is generally used used for purposes such as a feel modifier, a moisturiser, an algefacient, or a preservative, and examples are an alcohol such as ethanol or isopropyl alcohol, a glycol such as propylene glycol, 1,3-butylene glycol, dipropylene glycol, or polyethylene glycol, and a glycerol such as glycerine, diglycerine, or polyglycerine; one or more of which may be used.

The water-soluble polymer, if present, is generally used for purposes such as a viscosity modifier or a feel modifier, and examples are guar gum, gellan gum, pectin, agar, sodium chondroitin sulphate, hyaluronic acid, gum Arabic, sodium alginate, carrageenan, xanthan gum, locust bean gum, methylcellulose, hydroxyethylcellulose, carboxymethylcellulose, carboxyvinyl polymer, alkyl-modified carboxyvinyl polymer, polyvinyl alcohol, polyvinylpyrrolidone, or sodium polyacrylate; one or more of which may be used. Selecting an alkyl-modified carboxyvinyl polymer as the water-soluble polymer can obtain a cosmetic composition excellent in powder dispersibility and emulsion stability. The content in the case that the composition of the present invention contains a water-soluble polymer generally, is preferably 0.01-5 wt%, based on the weight of the composition.

In some cases, it has proved to be advantageous to adjust the viscosity of the compositions in accordance with the present invention to a value similar to the viscosity of Carnauba wax compositions, especially when used for cosmetic applications.

The mixture of dialiphatic ketones in the compositions of the present invention leads to a property profile which is particularly suitable for cosmetic applications.

One of the properties important for cosmetic application is the texture of the product.

Texture for the purpose of the present invention is an indicator of the resistance the product shows against compression respectively bending forces. Texture is expressed in grams or Newton and is descriptive of the load necessary to achieve a certain deformation. The higher the texture value in grams, the harder the product, i.e. the higher the force which is needed to achieve a certain compression or deformation. For cosmetic applications such as lipsticks, a texture in the range from 100 to 270 g, preferably in the range of from 120 to 250 g has shown to be advantageous.

In the course of the present invention, it has been found that ketone mixtures M with a certain ratio of ketones K1, K2 and K3 as defined hereinbefore provide better texture values than single ketones or most of binary blends of dialiphatic ketones, allowing to get a texture in the preferred range of from 120 g to 250 g. When blended with either stearone or laurone in specific ratii as to yield a ketone mixture M in accordance with the present invention, a ketone composition IK C₂₃-C₃₅ as used in the working examples and preferably obtained by decarboxylative carbonization leads to texture values exceeding what would have been expected , i.e. there is a synergism as far as texture is concerned, when IK C₂₃-C₃₅ is blended with either laurone or stearone to obtain a mixture ratio of ketones of type K1, K2 and K3 as in the mixtures of the present invention.

Another parameter of importance for cosmetic compositions is the softening point of the composition. Softening point should be at least appr. 45°C, preferably at least 65°C, more preferably at least 70°C to provide storage stability at elevated temperatures. If the softening point is below 45°C, there is a significant risk of failure of the product. On the other hand, softening pounts exceeding 90°C are not desirable as this might lead to difficulties in the application of the cosmetic product. In the course of the present invention it has been found that ketone mixtures M with a certain ratio of ketones K1, K2 and K3 as defined hereinbefore allow to get the softening point within the preferred range of from 65°C to 90°C.

The ketone mixtures M of the present invention lead to performing results in the sensory analysis. Sensory analysis for the purpose of the present invention means an evaluation of melting properties, sliding properties and deposition of material properties by a panel of experienced test persons. Scores are provided for the individual properties. It has been found that ketone mixtures M with a certain ratio of ketones K1, K2 and K3 as defined hereinbefore, allow to get desirable scores of at least 18 for each of the parameters.

Stability analysis is another parameter of importance for cosmetic compositions. The stability analysis provides a score from 1 to 5 for the properties of crystallization, volute, crack, uniformity and exudation at three different temperatures (-4°C, ambient temperature and 45°C). Measurements are conducted after 28 days and after two months. For practical applications the stability score should be preferably of at least 3.50 after two months, more preferably of at least 3.75 and still more preferably of at least 3.90.

In the course of the present invention, it has been found that that ketone mixtures M with a certain ratio of ketones K1, K2 and K3 as defined hereinbefore, allow to get a preferred score of at least 3.50, which can sometimes reach about 3.80 (as in example 8) or even the outsdanding value of about 4.00 (as in example 5).

Finally, it has been found, that the ketone mixtures M of the present invention can also be blended with natural waxes without loss in properties.

Thus, the present invention offers econimically feasible ketone mixtures M of dialiphatic ketones without loss in the performance properties of the product in cosmetic applications.

The compositions of the present invention are suitable and useful for the manufacture of a wide variety of cosmetic products. Just by way of example, color lipsticks, lip balms, lip glosses, lip pencils, foundations, mascaras, eyebrows, eyeshadows, eyeliners, skin colors, deodorant sticks, antiperspirant sticks, depilatory compositions, creams and other products for skin or hair care may be mentioned here.

The following examples show the effectiveness of the present invention.

### Components used:

IK C₂₃-C₃₅: A mixture of dialiphatic ketones obtained from decarboxylative ketonization of a coconut C₁₂-C₁₈ fatty acid cut comprising appr. 55 wt% of C₁₂-fatty acid, appr. 20 % of C₁₄ aliphatic fatty acid, and appr. 12 wt% of each of C₁₆ and C₁₈ aliphatic fatty acid in accordance with the process described in EP 3 292 097 and WO 2018/087179. The final product contained appr. 28 wt% of laurone (12 tricosanone), 22 wt% of C₂₅-aliphatic dialiphatic ketones (12-pentacosanone), 17 % of C₂₇-aliphatic dialiphatic ketones (12- and 14-heptacosanone), 18% of C₂₉-aliphatic dialiphatic ketones (12- and 14-nonacosanone), appr. 6 wt% of C₃₁ dialiphatic ketones (14- and 16-hentriacontanone), 3 % of C₃₃-dialiphatic ketones (16-tritriacontanone) and appr. 1,5% of C₃₅ dialiphatic ketones (18-pentatriacontanone or stearone).
Stearone (18-pentatriacontanone)
Laurone (12-tricosanone)

### Reference composition

For reference purposes a composition was prepared which comprised the following components and which resembles the properties of commercially available products:

### Wax components:

| | |
|---|---|
| Beeswax (Cerabio from Baerlocher) | 11 wt% |
| Candelilla wax (Candelilla blanche PAS from Baerlocher) | 3 wt% |
| Carnauba wax (Cerauba bio from Baerlocher) | 2 wt% |

Other components of the reference composition:

| | |
|---|---|
| C₁₀-C₁₈ triglycerides (Lipocire A SG (Gattefosse) | 13.8 wt% |
| Hydrostearic/Linoleic/Oleic Polyglycerides (Viamerine WH60 from Aldivia) | 3.0 wt% |
| Silica SB-700 (Miyoshi) | 5,0 wt% |
| Ricinus Communis Oil/Iron oxides Cl 77491 50/50 (COD 80006 from Maprecos) | 15,0 wt% |
| Jojoba seed oil (Aldivia) | 3.0 wt% |
| Sunflower seed oil (Aldivia) | 19.0 wt% |
| Castor seed oil (Chimie plus) | 25.0 wt% |
| Tocopherol/Sunflower seed oil (Dermofeel Toco 70, Dr. Straetmans) | 0.2 wt % |

The comparative composition showed a texture of 176 g and a softening point of 55.9°C.

The comparative composition contained a total of 16 wt% of waxes. In the compositions of the present invention, the wax components of the comparative composition were changed as indicated hereinafter. The remaining components are identical in the reference composition and the compositions in accordance with the present invention.

### Tests performed

Measurement of texture: Lipstick hardness (texture) was determined by measuring the peak load (in grams) with a Brookfield CT3 Texture analyzer. The measurement was carried out at ambient temperature within 24 to 96 hours after stick molding. The lipstick was mounted horizontally on the Texture Analyzer, then a metal yarn (TA 53 cutting wire, 0.33mm diameter, 40 mm length) was moved vertically through the stick at a speed of 1 mm/s and at a distance of 1 cm from the capsule containing the stick. The average of six measurements was taken.

Measurement of softening point: The ball ring method based on EN1427 was used to determine the softzening point. A brass ring with standard dimensions was filled with the composition and placed onto a holed support. A steel ball (9.5 mm diameter, weight 3.5 +/- 0.05 g) was deposited at the center of the tablet thus obtained. The support frame was immersed in a Pyrex beaker containing distilled water until the liquid level was 5 mm above the immersed ring and the water temperature was raised progressively. The softening point was taken as the temperature at which the ball touched the lower part of the support frame after going through the lipstick tablet. Two ball ring systems were used in parallel during one measurement and the average of the two measurements was taken.

Sensory analysis: Melting behaviour, sliding behaviour and deposition behaviour was evaluated by a test panel of six experienced persons giving scores for each parameter. Scores of at least 18 for each of the parameters are desirable.

Stability analysis: The lipstick stability was visually determined after a storage time of two months at 4°C, at ambient temperature and at 45°C of the closed lipstick. The following defects were inspected and rated from 1 to 5 (a score of 5 indicating a defect free product): crystallization, volutes, cracks, non-uniformity, exudation. The stability value is given as the geometric mean of the 5 items at the three different temperatures.

### Examples 1-11

The respective components were mixed and then shaped to form a lipstick for the measurements.

In Table 1, wax components of the compositions tested are listed and Table 2 shows the properties measured in accordance with the methods described above.

Examples with a "C" label are only provided for performance comparison with the examples in accordance with the invention (without "C" label). In no way, the presence of the "C" label should be construed as an admission from the Applicant that the corresponding examples form part of the state of the art. Even to the contrary, some exemplified ketone mixtures labelled with "C" might be well new, but do not meet the outstanding balance of properties achieved by the ketone mixtures in accordance with the present invention.

**Table 1**

| Example | Stearone wt% | Laurone wt% | IK C₂₃-C₃₅ wt% | Ketone K1 wt% | Ketone K2 wt% | Ketone K3 wt% |
|---|---|---|---|---|---|---|
| 1C | 0 | 0 | 0 | 0 | 0 | 0 |
| 2C | 16 | 0 | 0 | 0 | 0 | 100 |
| 3C | 0 | 16 | 0 | 100 | 0 | 0 |
| 4C | 8 | 8 | 0 | 50 | | 50 |
| 5 | 8 | 0 | 8 | 28.7 | 19.4 | 51.9 |
| 6C | 0 | 8 | 8 | 72,5 | 25,5 | 2,0 |
| 7C | 2.7 | 2.7 | 10.6 | 55.0 | 27 | 18 |
| 8 | 10.6 | 2.7 | 2.7 | 26.8 | 6.9 | 66.3 |
| 9 | 5.33 | 5.33 | 5.33 | 52,5 | 13.0 | 34.5 |
| 10C | 2.7 | 10.6 | 2.7 | 76.1 | 6.9 | 17.0 |
| 11C | 0 | 0 | 16 | 57,5 | 38,7 | 3.8 |

**Table 2**

| Ex. | Peak load (g) | Softening point (°C) | Melting | Sliding | Deposition | Stability 2 months |
|---|---|---|---|---|---|---|
| 1C | 176 | 56 | - | - | - | - |
| 2C | 67 | 80 | 25 | 23 | 22 | 4.35 |
| 3C | 93 | 57 | 17 | 23 | 18 | 3.19 |
| 4C | 149.5 | 71 | 18 | 19 | 20 | 3.0 |
| 5 | 184 | 72 | 20 | 19 | 18 | 3.97 |
| 6C | 172 | 48.5 | 24 | 22 | 24 | 3.35 |
| 7C | 220 | 51 | 23 | 24 | 24 | 3.84 |
| 8 | 155 | 74.5 | 22 | 24 | 24 | 3.79 |
| 9 | 179 | 66.7 | 22 | 23 | 22 | 3.57 |
| 10C | 223 | 56.5 | 18 | 18 | 18 | 3.83 |
| 11C | 141 | 47 | 30 | 30 | 30 | 3.84 |

The results show that compositions comprising the ketone mixtures M in accordance with the present invention provide an outstanding balance of properties. Stearone (Example 2C) or laurone (Example 3C) alone are inferior ; laurone (3C) is further inferior as concerns the softening point and the long term stability. A binary mixture of stearone and laurone (Example 4C) shows also inferior long term stability. Mixture 6C is also inferior in long term stability and exhibits further a poor softening point. Mixtures 7C, 10C and 11 C exhibit a poor softening point.

## Claims

1. A ketone mixture M comprising different dialiphatic ketones as follows
a) from 25 to 73 wt%, based on the total weight of ketone mixture M, of at least one dialiphatic ketone K1 comprising from 15 to 25 carbon atoms,
b) from 2 to 40 wt%, based on the total weight of ketone mixture M, of at least one dialiphatic ketone K2 comprising from 26 to 32 carbon atoms and,
c) from 25 to 90 wt%, based on the total weight of ketone mixture M, of at least one dialiphatic ketone K3 comprising from 33 to 47 carbon atoms.

2. The ketone mixture M in accordance with claim 1 wherein the dialiphatic ketones are **characterized by** the formula R-C(=O)-R' wherein R and R', which may be the same or different, represent a saturated or unsaturated, straight chain or branched aliphatic radical with of from 5 to 24 carbon atoms.

3. The ketone mixture in accordance with claim 1 or 2 wherein the dialiphatic ketone K1 comprises from 21 to 25 carbon atoms and is of formula R¹-C(=O)-R^{'1}, wherein R¹ and R^{'1} are linear C₁₁-C₁₃-alkyl.

4. The ketone mixture M in accordance with any of claims 1 to 3 wherein dialiphatic ketone K2 is of formula R²-C(=O)-R^{'2} wherein R² and R^{'2} are linear C₁₂-C₁₈ alkyl.

5. The ketone mixture M in accordance with any of claims 1 to 4 wherein dialiphatic ketone K₃ comprises from 33 to 37 carbon atoms and is of formula R³-C(=O)-R^{'3} wherein R³ and R^{'3} are linear C₁₇-C₁₉ alkyl.

6. The ketone mixture M in accordance with any of claims 1 to 5 wherein the weight fraction of the dialiphatic ketone K2, based on the total weight of the ketone mixture M, is at least 3.5 wt%, preferably at least 5 wt%.

7. The ketone mixture M in accordance with any of claims 1 to 6 wherein the weight fraction of the dialiphatic ketone K3, based on the total weight of the ketone mixture M, is at least 40 wt%, preferably at least 45 wt%.

8. The ketone mixture M in accordance with any of claims 1 to 7 wherein the dialiphatic ketones K1, K2 and K3 constitute at least 95 wt%, preferably at least 98 wt% of the ketone mixture.

9. The ketone mixture M in accordance with any of claims 1 to 8 consisting of ketones K1, K2 and K3.

10. The ketone mixture M in accordance with any of claims 1 to 9 wherein the dialiphatic ketones are obtained by decarboxylative ketonization of a fatty acid, a fatty acid derivative or a mixture thereof in the liquid phase with a metal compound as catalyst, the term fatty acid derivative referring to anhydrides made by the condensation of 2 fatty acids or to esters made by the condensation of fatty acids with alcohols.

11. A composition comprising the ketone mixture M in accordance with any one of claims 1 to 10.

12. The composition according to claim 11 comprising the ketone mixture M of any of claims 1 to 10 and additionally one or more components selected from
d) animal oils, vegetable oils, or synthetic oils, or from solid oils, semisolid oils, liquid oils, or volatile oils,
e) hydrocarbons, in particular fluid paraffins
f) waxes,
g) esters,
h) alcohols,
i) silicones,
j) fluorine based oils, and
k) lanolin.

13. The composition of claim 12 comprising from 1 to 40 wt% of the ketone mixture M in accordance with any of claims 1 to 10.

14. Use of the ketone mixture M in accordance with any of claims 1 to 10 or the composition in accordance with any of claims 11 to 13 for cosmetics.

## Patentansprüche

1. Ketongemisch M, umfassend wie folgt verschiedene dialiphatische Ketone
a) 25 bis 73 Gew.-%, bezogen auf das Gesamtgewicht des Ketongemischs M, mindestens eines dialiphatischen Ketons K1 mit 15 bis 25 Kohlenstoffatomen,
b) 2 bis 40 Gew.-%, bezogen auf das Gesamtgewicht des Ketongemischs M, mindestens eines dialiphatischen Ketons K2 mit 26 bis 32 Kohlenstoffatomen und
c) 25 bis 90 Gew.-%, bezogen auf das Gesamtgewicht des Ketongemischs M, mindestens eines dialiphatischen Ketons K3 mit 33 bis 47 Kohlenstoffatomen.

2. Ketongemisch M nach Anspruch 1, wobei die dialiphatischen Ketone durch die Formel R-C(=O)-R' gekennzeichnet sind, wobei R und R', die gleich oder verschieden sein können, für einen gesättigten oder ungesättigten, geradkettigen oder verzweigten aliphatischen Rest mit 5 bis 24 Kohlenstoffatomen stehen.

3. Ketongemisch nach Anspruch 1 oder 2 , wobei das dialiphatische Keton K1 21 bis 25 Kohlenstoffatome umfasst und durch Formel R¹-C(=O)-R'¹ wiedergegeben wird, wobei R¹ und R'¹ für lineares C₁₁-C₁₃-Alkyl stehen.

4. Ketongemisch M nach einem der Ansprüche 1 bis 3, wobei das dialiphatische Keton K2 durch Formel R²-C(=O)-R^{'2} wiedergegeben wird, wobei R² und R^{'2} für lineares C₁₂-C₁₈-Alkyl stehen.

5. Ketongemisch M nach einem der Ansprüche 1 bis 4, wobei das dialiphatische Keton K₃ 33 bis 37 Kohlenstoffatome umfasst und durch die Formel R³-C(=O)-R^{'3} wiedergegeben wird, wobei R³ und R^{'3} für lineares C₁₇-C₁₉-Alkyl stehen.

6. Ketongemisch M nach einem der Ansprüche 1 bis 5, wobei der Gewichtsanteil des dialiphatischen Ketons K2, bezogen auf das Gesamtgewicht des Ketongemischs M, mindestens 3,5 Gew.-%, vorzugsweise mindestens 5 Gew.-% beträgt.

7. Ketongemisch M nach einem der Ansprüche 1 bis 6, wobei der Gewichtsanteil des dialiphatischen Ketons K3, bezogen auf das Gesamtgewicht des Ketongemischs M, mindestens 40 Gew.-%, vorzugsweise mindestens 45 Gew.-% beträgt.

8. Ketongemisch M nach einem der Ansprüche 1 bis 7, wobei die dialiphatischen Ketone K1, K2 und K3 mindestens 95 Gew.-%, vorzugsweise mindestens 98 Gew.-% des Ketongemischs ausmachen.

9. Ketongemisch M nach einem der Ansprüche 1 bis 8 bestehend aus Ketonen K1, K2 und K3.

10. Ketongemisch M nach einem der Ansprüche 1 bis 9, wobei die dialiphatischen Ketone durch decarboxylative Ketonisierung einer Fettsäure, eines Fettsäurederivats oder eines Gemisches davon in flüssiger Phase mit einer Metallverbindung als Katalysator erhalten werden, wobei sich der Begriff Fettsäurederivat auf Anhydride, die durch Kondensation von 2 Fettsäuren hergestellt wurden, oder auf Ester, die durch Kondensation von Fettsäuren mit Alkoholen hergestellt wurden, bezieht.

11. Zusammensetzung, umfassend das Ketongemisch M nach einem der Ansprüche 1 bis 10.

12. Zusammensetzung nach Anspruch 11, umfassend das Ketongemisch M nach einem der Ansprüche 1 bis 10 und zusätzlich eine oder mehrere Komponenten, die aus Folgenden ausgewählt sind:
d) tierischen Ölen, pflanzlichen Ölen oder synthetischen Ölen oder aus festen Ölen, halbfesten Ölen, flüssigen Ölen oder flüchtigen Ölen,
e) Kohlenwasserstoffen, insbesondere flüssigen Paraffinen,
f) Wachsen,
g) Estern,
h) Alkoholen,
i) Silikonen,
j) Ölen auf Fluorbasis und
k) Lanolin.

13. Zusammensetzung nach Anspruch 12, umfassend 1 bis 40 Gew.- % des Ketongemischs M nach einem der Ansprüche 1 bis 10.

14. Verwendung des Ketongemischs M nach einem der Ansprüche 1 bis 10 oder der Zusammensetzung nach einem der Ansprüche 11 bis 13 bei Kosmetika.

## Revendications

1. Mélange de cétones M comprenant différentes cétones dialiphatiques comme suit
a) de 25 à 73 % en poids, sur la base du poids total de mélange de cétones M, d'au moins une cétone dialiphatique K1 comprenant de 15 à 25 atomes de carbone,
b) de 2 à 40 % en poids, sur la base du poids total de mélange de cétones M, d'au moins une cétone dialiphatique K2 comprenant de 26 à 32 atomes de carbone et,
c) de 25 à 90 % en poids, sur la base du poids total de mélange de cétones M, d'au moins une cétone dialiphatique K3 comprenant de 33 à 47 atomes de carbone.

2. Mélange de cétones M selon la revendication 1, dans lequel les cétones dialiphatiques sont **caractérisées par** la formule R-C(=O)-R' dans laquelle R et R', qui peuvent être identiques ou différents, représentent un radical aliphatique saturé ou insaturé, à chaîne linéaire ou ramifié, ayant de 5 à 24 atomes de carbone.

3. Mélange de cétones selon la revendication 1 ou 2, dans lequel la cétone dialiphatique K1 comprend de 21 à 25 atomes de carbone et répond à la formule R¹-C(=O)-R'¹, dans laquelle R¹ et R^{'1} sont alkyle linéaire en C₁₁-C₁₃.

4. Mélange de cétones M selon l'une quelconque des revendications 1 à 3, dans lequel la cétone dialiphatique K2 répond à la formule R²-C(=O)-R'² dans laquelle R² et R^{'2} sont alkyle linéaire en C₁₂-C₁₈.

5. Mélange de cétones M selon l'une quelconque des revendications 1 à 4, dans lequel la cétone dialiphatique K₃ comprend de 33 à 37 atomes de carbone et répond à la formule R³-C(=O)-R^{'3} dans laquelle R³ et R^{'3} sont alkyle linéaire en C₁₇-C₁₉.

6. Mélange de cétones M selon l'une quelconque des revendications 1 à 5, dans lequel la fraction pondérale de la cétone dialiphatique K2, par rapport au poids total du mélange de cétones M, est d'au moins 3,5 % en poids, de préférence d'au moins 5 % en poids.

7. Mélange de cétones M selon l'une quelconque des revendications 1 à 6, dans lequel la fraction pondérale de la cétone dialiphatique K3, par rapport au poids total du mélange de cétones M, est d'au moins 40 % en poids, de préférence d'au moins 45 % en poids.

8. Mélange de cétones M selon l'une quelconque des revendications 1 à 7, dans lequel les cétones dialiphatiques K1, K2 et K3 constituent au moins 95 % en poids, de préférence au moins 98 % en poids du mélange de cétones.

9. Mélange de cétones M selon l'une quelconque des revendications 1 à 8, constitué par les cétones K1, K2 et K3.

10. Mélange de cétones M selon l'une quelconque des revendications 1 à 9, dans lequel les cétones dialiphatiques sont obtenues par cétonisation décarboxylative d'un acide gras, d'un dérivé d'acide gras ou d'un mélange de ceux-ci en phase liquide avec un composé métallique en tant que catalyseur, le terme dérivé d'acide gras se référant à des anhydrides obtenus par condensation de 2 acides gras ou à des esters obtenus par condensation d'acides gras avec des alcools.

11. Composition comprenant le mélange de cétones M selon l'une quelconque des revendications 1 à 10.

12. Composition selon la revendication 11, comprenant le mélange de cétones M de l'une quelconque des revendications 1 à 10 et en outre un ou plusieurs composants choisis parmi
d) des huiles animales, des huiles végétales, ou des huiles synthétiques, ou parmi des huiles solides, des huiles semi-solides, des huiles liquides, ou des huiles volatiles,
e) des hydrocarbures, en particulier des paraffines fluides
f) des cires,
g) des esters,
h) des alcools,
i) des silicones,
j) des huiles à base de fluor, et
k) la lanoline.

13. Composition de la revendication 12, comprenant de 1 à 40 % en poids du mélange de cétones M selon l'une quelconque des revendications 1 à 10.

14. Utilisation du mélange de cétones M selon l'une quelconque des revendications 1 à 10 ou de la composition selon l'une quelconque des revendications 11 à 13 pour des produits cosmétiques.
